# EUROPEAN PATENT APPLICATION

(11) **EP 2 559 450 A1**
(43) Date of publication of application: **20.02.2013**
(21) Application number: 11006836.8
(22) Date of filing: 19.08.2011
(51) Int. Cl.: A61M 25/00, A61M 25/10

(54) **Shaft for medical devices and catheter**

(71) Applicant: Abbott Laboratories Vascular Enterprises Limited, Dublin 2 (IE)
(72) Inventor: Müller, Manfred, 79787 Lauchringen (DE); Corcoran, Louise, 70567 Stuttgart (DE)
(74) Representative: Peters, Hajo

(57) **Abstract**

The application relates to a shaft (10) for medical devices comprising an inner tube (11) and an outer tube (12), wherein the inner tube (11) and the outer tube (12) extend concentrically with a common longitudinal axis from a proximal end (15) to a distal end (16) of the shaft (10), and a spiral wall (13) projecting radially outwards from the inner tube (11).
Further, the invention relates to a catheter having such a shaft (10).

## Description

The invention relates to a shaft for medical devices, preferably inflatable devices, and a catheter having such a shaft.

### Background of the invention

There are multi-lumen catheters known in the art, as for example from US 7,022,106 B2. In these catheters, an outer lumen can be used for inflating and deflating a medical appliance, such as a balloon of a balloon catheter, or stent delivery systems.

In view of the catheters known from the state of the art, there is potential for further increasing the inflation/deflation speed.

### Summary of the invention

It is an object of the present invention to provide a shaft for medical devices with improved inflation and/or deflation characteristics.

This object is solved with a shaft according to the independent claim. Advantageous further developments are subject of the dependent claims.

According to a first embodiment of the invention, there is provided a shaft for medical devices comprising an inner tube and an outer tube, wherein the inner tube and the outer tube extend concentrically with a common longitudinal axis from a proximal end to a distal end of the shaft, and a spiral wall projecting radially outwards from the inner tube. The advantage of the resulting spiral geometry of a lumen formed between the inner and the outer tube is to alter the flow characteristics of an inflation fluid guided through the inflation lumen. The spiral geometry reduces turbulent flow and promotes laminar flow. This way, the speed of deflation can be increased which can be advantageous in clinical applications.

Moreover, it can be beneficial that the spiral wall is monolithically formed with the inner tube. This provides an efficient manufacturing and a good durability.

Furthermore, the shaft can be formed such that the spiral wall is in contact with the inner tube and the outer tube. This way a tortuous curvature of the spiral wall is achieved. This can be beneficial as it ensures a more laminar flow of the inflation media and reduces the turbulent flow that might otherwise occur. Moreover, a connection between the inner and the outer tube is created, which improves the push efficiency or force transmission of the catheter system as it allows that a force applied to the outer tube by a user or physician can be transmitted to a tip of the catheter, which is attached to the inner tube, more optimally. Due to this improved transmission of force from the outer tube via the spiral wall to the inner tube and finally to the catheter tip, the maneuverability or deliverability of the catheter system in tortuous calcified anatomy can be improved.

Further, it can be advantageous that the inner tube, the outer tube and the spiral wall are monolithically formed. This leads to an easier production and a better durability.

According to a further development, the spiral wall has a rounded cross-section, and according to a yet further development, the spiral wall has a circular cross-section.

Moreover, the shaft can be made of polymeric material. This provides a flexible, shaft.

These and other embodiments are described in more detail with reference to the Figures.

### Brief description of the Figures

Fig. 1 is a three-dimensional view of a shaft according to a first embodiment of the invention;
Fig. 2 is a three-dimensional, transparent illustration of the shaft according to the first embodiment;
Fig. 3 is a longitudinal sectional view of the shaft according to the first embodiment;
Fig. 4 is a three-dimensional view of the shaft according to the first embodiment viewing the shaft in cross-section;
Fig. 5 is a three-dimensional view of a shaft according to a second embodiment of the invention, and
Fig. 6 is a longitudinal sectional view of the shaft according to the second embodiment of the invention.

### Detailed description of the invention

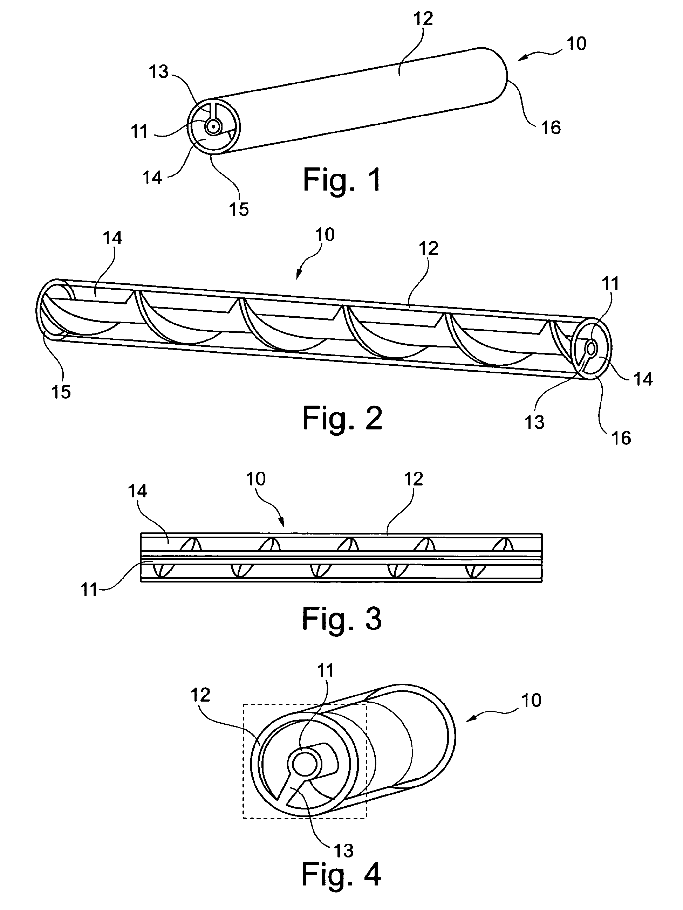

Figs. 1 to 4 show a shaft 10 according to a first embodiment of the invention, wherein Fig. 1 is a three-dimensional view, Fig. 2 is a three-dimensional, transparent illustration, Fig. 3 is a longitudinal sectional view, and Fig. 4 is a three-dimensional view showing the shaft 10 in cross-section. The shaft 10 comprises an inner tube 11 and an outer tube 12, the inner tube 11 being arranged inside the outer tube 12. The inner tube 11 and the outer tube 12 are flexible and preferably free of openings in a radial direction, respectively. The inner tube 11 and the outer tube 12 are arranged co-axially, i.e. they have a common longitudinal axis. When viewed in cross-section, a spiral wall 13 projects radially from an outer surface of the inner tube 11 to an inner surface of the outer tube 12. In a longitudinal direction of the shaft 10, the spiral wall 13 runs helically around the inner tube 11 with the common longitudinal axis of the tubes 11, 12 as center axis. Preferably, the inner tube 11, the outer tube 12 and the spiral wall 13 are formed monolithically of polymeric material. In more detail, the shaft is produced by twisting the shaft 10 about its longitudinal axis when it is extruded. Thus, a central inner tube 11 is formed which is surrounded by a spiral shaped inflation lumen 14 which is formed between the radially outer surface of the inner tube 11 and the radially inner surface of the outer tube 12. This inflation lumen 14 runs longitudinally through the entire length of the shaft 10, i.e. from a proximal end 15 of the shaft 10 to a distal end 16 of the shaft 10. The inside of the inner tube 11 is separated in a fluid-tight manner from the inflation lumen 14.

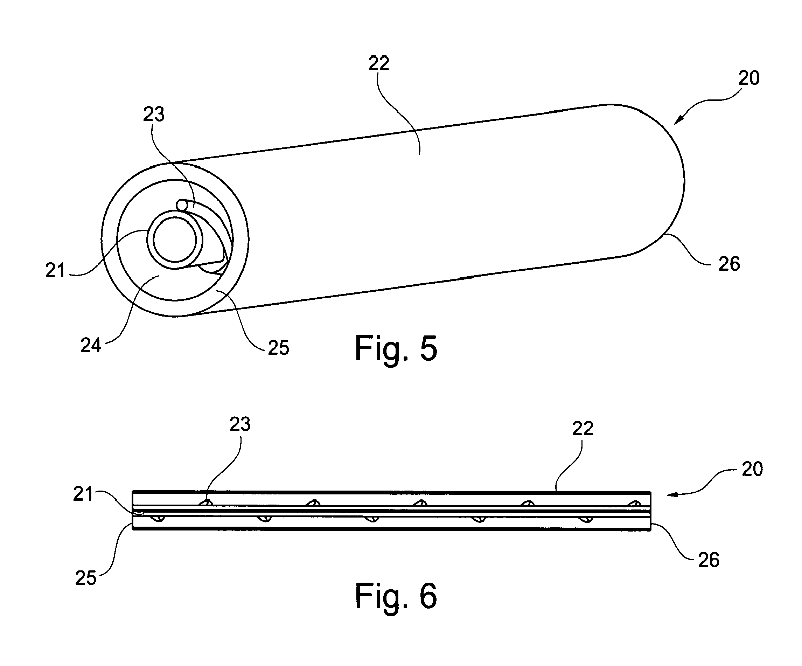

Figs. 5 and 6 show a shaft 20 according to a second embodiment of the invention, wherein Fig. 5 is a three-dimensional view, and Fig. 6 is a longitudinal sectional view. The shaft 20 comprises an inner tube 21 and an outer tube 22, the inner tube 21 being arranged inside the outer tube 22. The inner tube 21 and the outer tube 22 are flexible and preferably free of openings in a radial direction, respectively. Preferably, the inner tube 21 and the outer tube 22 are arranged co-axially. When viewed in cross-section, a spiral wall 23 projects radially from an outer surface of the inner tube 21 without contacting an inner surface of the outer tube 22. Also in cross-section of the shaft 20, the spiral wall 23 has a rounded cross-section, preferably a circular cross-section, the outer circumferences of the inner tube 21 and the spiral wall 23 overlap slightly in the area in which these elements are connected. In a longitudinal direction of the shaft 20, the spiral wall 23 runs helically around the inner tube 21 with the common longitudinal axis of the tubes 21, 22 as center axis. Preferably, the inner tube 21 and the spiral wall 23 are formed monolithically. The inner tube, the outer tube 22 and the spiral wall 23 are preferably made of polymeric material. The second embodiment of the invention allows, to manufacture the inner tube 21 with the spiral wall 23 formed on it separately from the outer tube. In more detail, the inner tube 21 is produced with the spiral wall 23 formed on it by twisting or rotating the inner tube 21 about its longitudinal axis when it is extruded. The outer tube 22 can simply be extruded without twisting. Thereafter, the inner tube 21 can be inserted into the outer tube 22. Thus, a central inner tube 21 is formed which is surrounded by a inflation lumen 24 which is formed between the radially outer surface of the inner tube 21 and the radially inner surface of the outer tube 22, wherein the inflation lumen 24 has a spiral wall influencing the flow characteristics. This inflation lumen 24 runs in a longitudinally direction through the entire length of the shaft 20, i.e. from a proximal end 25 of the shaft 20 to a distal end 26 of the shaft 20. The inside of the inner tube 21 is separated in a fluid-tight manner from the inflation lumen 24.

While the invention has been illustrated and described in detail in the drawings and foregoing description, such illustration and description are to be considered illustrative or exemplary and not restrictive and it is not intended to limit the invention to the disclosed embodiments. The mere fact that certain measures are recited in mutually different dependent claims does not indicate that a combination of these measures cannot be used advantageously.

## Claims

1. Shaft (10) for medical devices comprising
an inner tube (11) and an outer tube (12), wherein the inner tube (11) and the outer tube (12) extend concentrically with a common longitudinal axis from a proximal end (15) to a distal end (16) of the shaft (10), and
a spiral wall (13) projecting radially outwards from the inner tube (11).

2. Shaft (10) according to claim 1, wherein the spiral wall (13) is monolithically formed with the inner tube (11).

3. Shaft (10) according to claim 1 or 2, wherein the spiral wall (13) is in contact with the inner tube (11) and the outer tube (12).

4. Shaft (10) according to claim 3, wherein the inner tube (11), the outer tube (12) and the spiral wall (13) are monolithically formed.

5. Shaft (10) according to claim 1 or 2, wherein the spiral wall (13) has a rounded cross-section.

6. Shaft (10) according to claim 5, wherein the spiral wall (13) has a circular cross-section.

7. Shaft (10) according to anyone of the preceding claims, wherein the shaft (10) is made of polymeric material.

8. Catheter having a shaft (10) according to anyone of the preceding claims.
